# EUROPEAN PATENT APPLICATION

(11) **EP 2 112 513 A2**
(43) Date of publication of application: **28.10.2009**
(21) Application number: 09155826.2
(22) Date of filing: 23.03.2009
(51) Int. Cl.: G01N 33/543, G01N 33/558

(54) **Chromatographic test device**

(30) Priority: 27.03.2008 JP 2008083171
(71) Applicant: Sysmex Corporation, Kobe-shi, Hyogo 651-0073 (JP)
(72) Inventor: Manabe, Tomoko, Hyogo 651-0073 (JP)
(74) Representative: Paemen, Liesbet R.J.

(57) **Abstract**

A chromatographic test device comprising, in the order of passage of a sample containing blood cell components and liquid components, a label holding member provided with a labeling substance capable of binding specifically to an analyte contained in the liquid components, a blood cell separation member for separating predetermined blood cell components in the sample from the liquid components, and a chromatographic carrier carrying a capturing substance capable of binding specifically to the analyte.

## Description

### FIELD OF THE INVENTION

The present invention relates to a chromatographic test device for detecting an analyte in a sample by using a substance capable of binding specifically to the analyte, which is used in a test conducted using whole blood.

### BACKGROUND

As a test device for detecting an analyte in a sample by using a substance binding specifically to the analyte, there is a chromatographic test device. Conventionally, such a chromatographic test device has been used to detect an analyte mainly in a serum sample, but in recent years, a chromatographic test device also used for whole blood has appeared.

For example, Japanese Laid-Open Patent Publication 2000-258418 discloses a chromatographic test device provided with a blood cell separation part through which blood cell components in whole blood are removed and only plasma components are allowed to pass. When a sample (whole blood) is added to this test device, only plasma components in the sample pass through the blood cell separation part and reach a reagent part, while red blood cells in the sample cannot pass through the blood cell separation part. Then, an analyte in the plasma is bound to a labeled antibody contained in the reagent part, to form a complex. The complex, together with the plasma components, is developed on a developing layer and captured in a measurement part. Since the captured complex has been labeled with the colored labeled antibody, the analyte in the sample can be detected by visually confirming the measurement part. In this test device, red blood cells in the sample cannot pass through the blood cell separation part, and thus the confirmation of the measurement part is not made difficult by the color of red blood cells.

However, as described in Japanese Laid-Open Patent Publication 2000-258418 supra, there is a problem that when the chromatographic test device having a blood cell separation part and a reagent part arranged in the order of passage of a sample is used, a labeled antibody is retained on a developing layer to cause an increase in background. In the chromatographic test device, an analyte labeled with a labeling substance is captured in a chromatographic carrier, and the label thereby appearing on a measurement part is confirmed with the naked eye, thereby judging the presence or absence of the analyte, and therefore, an increase in background leads to difficult discrimination of the label on the measurement part, to make judgment difficult. Particularly when an analyte is present in a trace amount, the label on the measurement part may become unclear so that if the background is high, the label cannot be accurately perceived, resulting sometimes in erroneous judgment.

### SUMMARY

The scope of the present invention is defined solely by the appended claims, and is not affected to any degree by the statements within this summary.

A first aspect of the present invention is a chromatographic test device which comprises, in the order of passage of a sample containing blood cell components and liquid components, a label holding member provided with a labeling substance capable of binding specifically to an analyte contained in the liquid components, a blood cell separation member for separating predetermined blood cell components in the sample from the liquid components, and a chromatographic carrier carrying a capturing substance capable of binding specifically to the analyte.

A second aspect of the present invention is a chromatographic test device which comprises, in the order of passage of a sample containing blood cell components and liquid components, a first blood cell separation member for separating predetermined blood cell components in the sample from the liquid components, a label holding member provided with a labeling substance capable of binding specifically to an analyte contained in the liquid components, a second blood cell separation member for further separating, from the liquid components, the blood cell components separated by the first blood cell separation member, and a chromatographic carrier carrying a capturing substance capable of binding specifically to the analyte.

A third aspect of the present invention is a chromatographic test device which comprises, in the order of passage of a sample containing blood cell components and liquid components, a first blood cell separation member for separating predetermined blood cell components from the liquid components, the first blood cell separation member being provided with a labeling substance capable of binding specifically to an analyte contained in the liquid components, a second blood cell separation member for further separating, from the liquid components, the blood cell components separated by the first blood cell separation member, and a chromatographic carrier carrying a capturing substance capable of binding specifically to the analyte.

### BRIEF DESCRIPTION OF THE DRAWINGS

FIG. 1 is a cross-sectional view showing a structure of a chromatographic test device in an embodiment of the present invention;
FIG. 2 is a cross-sectional view showing a structure of a chromatographic test device in Comparative Example 1;
FIG. 3 is a cross-sectional view showing a structure of a chromatographic test device in another embodiment of the present invention;
FIG. 4 is a cross-sectional view showing a structure of a chromatographic test device in another embodiment of the present invention;
FIG. 5 is a cross-sectional view showing a structure of a chromatographic test device in another embodiment of the present invention;
FIG. 6 is a cross-sectional view showing a structure of a chromatographic test device in another embodiment of the present invention;
FIG. 7 is a cross-sectional view showing a structure of a chromatographic test device in another embodiment of the present invention;
FIG. 8 is a photocopy of a photograph showing a developed state with time of a sample in each of the test devices in Example 1 and Comparative Example 1 wherein whole blood was used as the sample; and
FIG. 9 is a photocopy of a photograph showing a developed state with time of a sample in each of the test devices in Example 1 and Comparative Example 1 wherein serum was used as the sample.

### DETAILED DESCRIPTION OF THE PREFERRED EMBODIMENTS

The preferred embodiments of the present invention are described hereinafter with reference to the drawings.

Hereinafter, embodiments of the chromatographic test device of the present invention are described in detail by reference to the drawings.

### 1. Constitution of the chromatographic test device

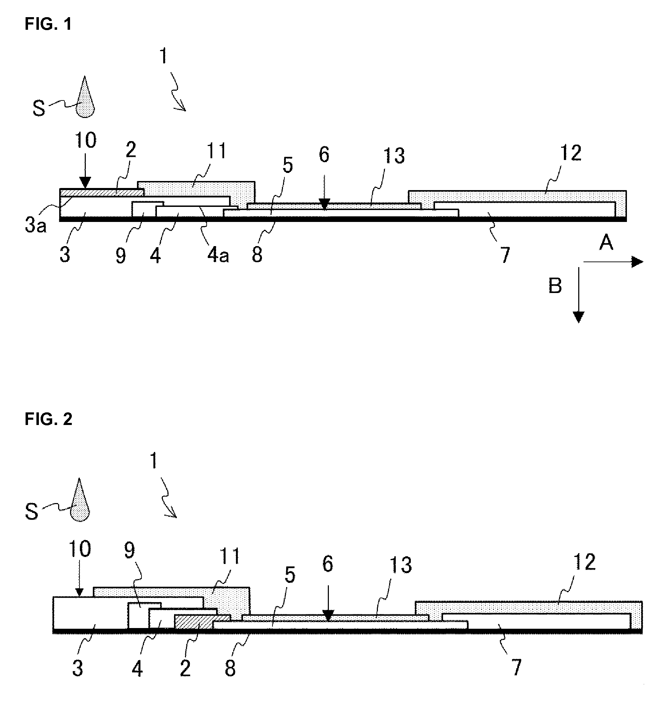

FIG. 1 is a cross-sectional view showing the structure of a chromatographic test device (also referred to hereinafter as "test device") in an embodiment of the present invention. As shown in FIG. 1, a test device 1 is constituted mainly by including a label holding member 2, a first blood cell separation member 3, a second blood cell separation member 4, a chromatographic carrier 5 and an absorbent member 7 in the order of passage of a sample S added.

The sample S is analyzed with the test device in this embodiment to examine whether an analyte is contained in the sample S. As long as the sample S contains blood cell components and liquid components, the sample S may be blood itself, a dilution of blood in a developing solvent, or blood treated to remove a part of its blood components. The blood cell components include, for example, red blood cells, white blood cells and platelets. When the sample S is blood itself, the liquid components comprise plasma in blood. When the sample S is a dilution of blood in a developing solvent, the liquid components are composed of blood plasma and the developing solvent.

Generally, the chromatographic device is constituted such that when a sample is added to a specific part (for example, a sample addition part 10 in FIG. 1), the added sample moves through members successively to appear on a chromatographic carrier, followed by development (movement) on the chromatographic carrier to reach a judgment part 6. In this specification, the direction (indicated by arrow A in FIG. 1) in which the sample is developed on the chromatographic carrier in the test device 1 set horizontally as shown in FIG. 1 is referred to as "direction of sample development", and the direction (indicated by arrow B in FIG. 1) perpendicular to the direction of sample development is referred to as "vertical direction".

The label holding member 2 has (contained therein but free to pass through it when in liquid) a labeling substance capable of binding specifically to an analyte. The first blood cell separation member 3 is constituted so as to allow liquid components contained in the sample S to move faster than predetermined blood cell components such as red blood cells and white blood cells, thereby separating the predetermined blood cell components from the liquid components. The second blood cell separation member 4 is constituted so as to capture the blood cell components and pass the liquid components, thereby separating the predetermined blood cell components from the liquid components. A chromatographic carrier 5 carries, on a judgment part 6, a capturing substance capable of binding specifically to an analyte.

As shown in FIG. 1, the label holding member 2 and the first blood cell separation member 3 are disposed so as to overlap vertically with each other in the vertical direction in a horizontally disposed state of the test device 1 and to be contacted with each other via a contact area or contact face 3a only in the vertical direction. The first blood cell separation member 3 and the second blood cell separation member 4 are disposed so as to be contacted with each other via a contact face 4a. A sealing member 9 for preventing the sample S from penetrating from the first blood cell separation member 3 to the second blood cell separation member 4 in the direction of sample development is disposed between the first blood cell separation member 3 and the second blood cell separation member 4. The second blood cell separation member 4 and the chromatographic carrier 5 are disposed so as to be contacted with each other both in the direction of sample development and in the vertical direction.

An absorbent member 7 is disposed on the sample development downstream side of the chromatographic carrier 5, so as to contact with the chromatographic carrier 5. The first blood cell separation member 3, the second blood cell separation member 4, the chromatographic carrier 5 and the absorbent member 7 are attached to a base material 8 consisting of, for example, plastics, paper or glass. The test device 1 is covered with protective sheets 11 to 13.

Hereinafter, the respective constituent members are described in detail.

### Label holding member 2

The label holding member 2 is a member containing a labeling substance capable of binding specifically to an analyte potentially present or contained in liquid components in the sample S. The label holding member 2 is disposed in the position through which the sample S passes first in the test device 1, and has a sample addition part 10 to which the sample S is added.

As used herein, the analyte (the substance to be detected) is not particularly limited with respect to its type as long as it is contained in blood such as human blood and not filtered out with the second blood cell separation member 4 described later. Examples of the analyte include antigens, antibodies, hormones, hormone receptors, lectins, lectin-binding carbohydrates, drugs or metabolites thereof, drug receptors, nucleic acids, and fragments, variants and combinations thereof. In more detail, examples of the analyte include cells such as bacteria cells, protist cells and mycete cells, viruses, proteins and polysaccharides, which can be exemplified by influenza viruses, parainfluenza virus, RS virus, Mycoplasma pneumoniae, rotavirus, calicivirus, coronavirus, adenovirus, enterovirus, herpes virus, human immunodeficiency virus, hepatitis virus, pathogenic viruses causing severe acute respiratory syndrome, Escherichia coli, Staphylococcus aureus, Streptococcus pneumoniae, Streptococcus pyogenes, malaria parasite, other pathogens causing various diseases such as digestive system disease, central nervous system diseases and hemorrhagic fever, metabolites thereof, tumor markers such as carcinoembryonic antigen and CYFRA, and hormones.

When canine blood is used, the analyte is further exemplified by filarial worm antigens. When feline blood is used, the analyte is further exemplified by feline leukemia virus, feline immunodeficiency virus and antibodies thereof.

The labeling substance contained in the label holding member 2 is a substance capable of binding specifically to the analyte. For example, the labeling substance may be one wherein a substance capable of specifically binding to the analyte is labeled with a colored particle or colored particles.

For example, when the analyte is an antigen or antibody, the substance capable of binding specifically to the analyte may be an antibody or antigen capable of binding specifically to the antigen or antibody via antigen-antibody reaction. Examples of the analyte include substances which, based on biological affinity such as ligand-receptor bonding, can bind specifically to the analyte.

The colored particles include, for example, colored latex particles, metallic colloids such as gold colloids.

When a substance capable of specifically binding to the analyte is thus labeled with colored particles and then used as the labeling substance, the presence or absence of the analyte in the sample can be confirmed with the naked eye.

In the label holding member 2, various materials such as nonwoven fabrics and porous members can be used, and particularly porous members such as glass fibers and cellulose fibers are preferably used. When a porous member is used as the first blood cell separation member 3 described later, the pore size of a porous member used as the label holding member 2 is preferably larger than the pore size of the first blood cell separation member 3. By this structure, a sample easily permeates into the label holding member 2, and the sample spreads sufficiently in the label holding member 2 and then moves to the first blood cell separation member 3, thus improving the efficiency of development. The pore size of the label holding member 2 is larger than the pore size of the first blood cell separation member 3 so that the sample permeates sufficiently into the label holding member 2 and then moves gradually to the first blood cell separation member 3, thus prolonging the time of contact of the sample with the label holding member 2 to achieve more sufficient elution of the labeling substance.

Preferably, the label holding member 2 is disposed so as to overlap vertically with the first blood cell separation member 3 described later in the vertical direction. By this structure, the majority of the sample S that has permeated into the label holding member 2 moves by its own weight to the first blood cell separation member 3, and thus the amount of the sample finally reaching the judgment part 6 in the chromatographic carrier 5 can be increased to enable more accurate detection of the analyte.

### First blood cell separation member 3

The first blood cell separation member 3 is a member for separating blood cell components such as red blood cells and white blood cells from liquid components contained in the sample S by moving the liquid components faster than the blood cell components.

Specifically, the first blood cell separation member 3 consists of a porous member and its pore size is equal in size to or slightly larger than blood cells (particularly red blood cells). When such a member is used, the liquid components can move smoothly in the pores, while the predetermined blood cell components such as red blood cells and white blood cells cannot move smoothly in the pores, and thus the liquid components migrate more rapidly than the blood cell components in the first blood cell separation member 3. Accordingly, the blood cell components are separated from the liquid components.

Blood cells contained in blood vary in size depending on their type. Therefore, the sample can contain blood cell components of various sizes. For example, when the sample contains human blood, the blood cells that can be contained in the sample and their sizes are as follows: among leukocytes, lymphocytes are 6 to 15 µm in size, monocytes are 12 to 18 µm, neutrophils are 10 to 12 µm, basophils are 10 to 15 µm and eosinophils are 10 to 15 µm, and red blood cells are 7 to 8 µm and platelets are 1 to 4 µm. Blood cell components of relatively small size such as platelets might move in the first blood cell separation member 3 at almost the same speed as that of blood cell components of relatively large size such as red blood cells. However, even if platelets appear on the judgment part 6, judgment on the judgment part 6 is not made difficult by the color of platelets, and the influence of platelets on detection results is so low that the working effect of this embodiment is not significantly influenced.

The size of blood cells such as red blood cells varies depending on the type of animal from which the blood cells are derived. For example, canine red blood cells and feline red blood cells are respectively about 80% and about 50% in size relative to human red blood cells. Therefore, the size of pores in the second blood cell separation member 4 is suitably selected depending on the size of red blood cells of the animal type from which blood used as a sample is derived.

The first blood cell separation member 3 can make use of a membrane used in paper chromatography or thin-layer chromatography. Examples of such usable membrane are membranes made of various materials such as nitrocellulose, nylon (for example, modified nylon into which a carboxyl group, and an amino group which may be substituted with an alkyl group, have been introduced), polyvinylidene difluoride (PVDF) and cellulose acetate. Specifically, commercial products such as MF1 (lateral flow type, Whatman), LF1 (lateral flow type, Whatman) and FUSION5 (Whatman) may be used. The first blood cell separation member 3 is preferably in the form of a membrane that transfers the sample in the direction of sample development, to separate the sample in the direction of sample development, wherein the structure of the first blood cell separation member 3, and the direction in which the sample is separated by the first blood cell separation member 3, are not particularly limited. For example, the first blood cell separation member 3 may be in the form of a block in which the sample is separated in the vertical direction.

A sealing member 9 for preventing the infiltration of the sample from the first blood cell separation member 3 to the second blood cell separation member 4 in the direction of sample development is disposed between the first blood cell separation member 3 and the second blood cell separation member 4. As long as the sealing member 9 can prevent the infiltration of the sample, its constitution and material are not particularly limited. Specifically, commercial products such as ARcare series (Adhesives Corporation) may be used as the sealing member.

In this embodiment, the first blood cell separation member 3 and the second blood cell separation member 4 are constituted so as to be directly contacted with each other via the contact face 4a, but another member not preventing the movement of the sample from the first blood cell separation member 3 to the second blood cell separation member 4 may be disposed between the first blood cell separation member 3 and the second blood cell separation member 4.

### Second blood cell separation member 4

The second blood cell separation member 4 is a member for separating blood cell components such as red blood cells and white blood cells from liquid components by capturing red blood cells and simultaneously allowing the liquid components to pass. The second blood cell separation member 4 is disposed on the sample development downstream side of the first blood cell separation member.

By way of example, the second blood cell separation member 4 consists of a porous member having pores smaller than blood cells (for example, red blood cells) to be captured. By this structure, blood cells to be captured cannot pass through the pores and are captured by the second blood cell separation member 4. The size of pores in the porous member may be decreased in the direction going from the upper to the lower surfaces of the porous member (in horizontal position of the device). In this case, pores at the side of the upper surface of the porous member are relatively large to allow the sample to spread easily, and the sample arrives, in a sufficiently spread state, at pores of relatively small size at the side of the lower surface of the porous member, and the blood cell components are captured by these pores, thus enabling efficient separation.

By arranging the second blood cell separation member 4, the liquid components in the sample are first discharged from the first blood cell separation member and then pass through the second blood cell separation member, and the predetermined blood cell components in the sample arrive, later than the liquid components, at the second blood cell separation member. It follows that prior to clogging of the second blood cell separation member with the predetermined blood cell components in the sample, the liquid components pass through the second blood cell separation member, and therefore, the liquid components are smoothly discharged into the chromatographic carrier, to enable rapid examination.

The second blood cell separation member 4 can make use of a commercially available filtration membrane. The commercially available filtration membrane includes, for example, BTS SP 100, BTS SP 200 and BTS SP 300 (Vertical Flow Type, PALL Corporation). The second blood cell separation member 4 is preferably in the form of a membrane which transfers the sample in the vertical direction, to separate the sample in the vertical direction. The constitution of the second blood cell separation membrane 4, and the direction in which the sample is separated by the second blood cell separation member 4, are not particularly limited. For example, the second blood cell separation member 4 may be in the form of a block which separates the sample in the direction of sample development.

### Chromatographic carrier 5, judgment part 6

The chromatographic carrier 5 is a member that carries (e.g. immobilized), on the judgment part 6, a capturing substance capable of binding specifically to an analyte.

The capturing substance may be that which binds specifically to an analyte. The specific bond between the capturing substance and the analyte includes, for example, bonds based on biological affinity such as a bond due to an antigen-antibody reaction and a ligand-receptor bond.

For example, when the analyte is an antigen, antibodies against the antigen can be used as the labeling substance and the capturing substance. In this case, an antibody used as the labeling substance and an antibody used as the capturing substance are preferably those recognizing different sites (epitope) of the antigen respectively.

For example, when the analyte is an antibody, an antigen against the antibody can be used as the capturing substance, and a substance (antibody) capable of binding specifically to the antibody can be used as the labeling substance.

The chromatographic carrier 5 can make use of a variety of materials such as nitrocellulose, nylon (for example, modified nylon into which a carboxyl group, and an amino group which may be substituted with an alkyl group, have been introduced), polyvinylidene difluoride (PVDF) and cellulose acetate. The absorbent member 7 can make use of a variety of materials such as cellulose and glass fiber.

### Absorbent member 7

The absorbent member 7 is a member for accelerating the development of a sample by absorbing the liquid components in the sample that has passed through the chromatographic member 5, and is disposed on the sample development downstream side of the chromatographic carrier 5. The absorbent member 7 can use, for example, a nonwoven fabric or a porous member and is not particularly limited as long as it is a member capable of absorbing liquid components by capillary phenomenon. The absorbent member 7 may be omitted. When the absorbent member 7 is omitted, the chromatographic carrier 5 may be made longer to accelerate development of the sample.

### Protective sheets 11, 12, 13

The test device 1 in accordance with this embodiment is provided with a protective member (for example, protective sheets 11, 12 and 13) for preventing the evaporation of liquid components contained in the sample S. The protective sheet 11 is disposed on the sample development upstream side of the test device 1, and the protective sheet 11 disposed on the upstream side covers the end of the downstream side of the labeling holding member 2, the first blood cell separation member 3 and the second blood cell separation member 4. The protective sheet 12 is disposed on the sample development downstream side of the test device 1 and covers the absorbent member 7. The protective sheet 13 is disposed between the protective sheet 11 and the protective sheet 12, and covers the chromatographic carrier 5 including the judgment part 6.

The protective sheets 11 and 12 disposed on the sample development upstream side and downstream side respectively may be identical members. The protective sheet 13 for covering the chromatographic carrier 5 is preferably a transparent or semitransparent member so as not to prevent the visibility of the judgment part 6. By providing the test device with the protective sheets 11, 12 and 13, it is possible to prevent the evaporation of liquid components contained in the sample, thereby improving the efficiency of development of the sample. The protective sheets 11, 12 and 13 may be composed of one member that does not disturb the visibility of the judgment part 6.

### 2. Method of using the chromatographic test device, and the like

Hereinafter, the method of using a test device 1 in an embodiment of the present invention and the working of the test device 1 are described in detail with reference to FIG. 1. The direction in which the test device 1 is disposed at the time of examination is not particularly limited. The test device 1 may be used either in a horizontally disposed state or in a vertically disposed state. Hereinafter, as an example, the test device 1 in the case of being used in a horizontally disposed state is described.

### (1) Addition of sample S

First, the sample S is added to the sample addition part 10. At this point, it is not evident whether an intended analyte is contained in the sample S or not. In the following description, it is assumed that the analyte is contained in the sample S. As the sample S, Whole blood is used.

The sample addition part 10 is the part of the test device to which the sample is added. The sample addition part 10 refers to a site that is assigned, by manufacture's instructions or by an indication on the test device 1 itself or on a container accommodating the test device 1, as the site to which the sample is added. In the test device 1 in this embodiment, the sample addition part 10 is disposed on the sample development upstream side of the label holding member 2.

The sample S that has been added to the sample addition part 10 permeates into the label holding member 2 and migrates, by capillary phenomenon, in the first blood cell separation member 3, the second blood cell separation member 4, the chromatographic carrier 5 and the absorbent member 7 in this order.

### (2) Formation of a complex

The sample S that has been added to the sample addition part 10 permeates into the label holding member 2 and moves in the label holding member 2 by capillary phenomenon while eluting a labeling substance held with the label holding member 2. The label holding member 2 has a labeling substance capable of binding specifically to an analyte. Accordingly, when the analyte is contained in the sample S, the labeling substance binds specifically to the analyte to form a complex. The formed complex, together with the liquid components contained in the sample S, moves in the label holding member 2 and moves in the vertical direction to the first blood cell separation member 3 that was disposed so as to overlap vertically with the label holding member 2. The analyte and the labeling substance, both of which are still in a form not forming the complex (that is, in a state suspended in the liquid components), also move together with the sample S to the first blood cell separation member 3.

### (3) Separation with the first blood cell separation member

The sample S that has passed through the label holding member 2 infiltrates the first blood cell separation member 3 where the sample S is separated chromatographically. The first blood cell separation member 3 is constituted so as to move the liquid components contained in the sample S faster than the blood cell components, thereby separating the blood cell components from the liquid components, and thus the liquid components in the sample S are initially discharged from the first blood cell separation member 3. The blood cell components in the sample S are discharged later than the liquid components, from the first blood cell separation member 3.

The sample S discharged from the first blood cell separation member 3 passes through the area of contact of the first blood cell separation member 3 with the second blood cell separation member 4 and migrates to the second blood cell separation member 4. In this embodiment, the first blood cell separation member 3 and the second blood cell separation member 4 are provided therebetween with a sealing member 9 for preventing the infiltration of the sample S to the second blood cell separation member 4 in the direction of sample development. In this structure, the first blood cell separation member 3 and the second blood cell separation member 4 are contacted with each other via the contact face 4a only in the vertical direction, and thus the sample S discharged from the first blood cell separation member 3 infiltrates the second blood cell separation member 4 in the vertical direction. In this embodiment, the second blood cell separation member 4 for separating blood cell components is constituted so as to separate the blood cell components in the vertical direction as described later. Hence, the sealing member 9 can be disposed as described above to regulate the direction in which the sample S infiltrates the second blood cell separation member 4, thereby attaining more suitable separation of the blood cell components with the second blood cell separation member 4.

### (4) Separation with the second blood cell separation member

The sample S that has transferred to the second blood cell separation member 4 moves in the second blood cell separation member 4 in the vertical direction. The second blood cell separation member 4 separates the sample S via filtration. The blood cell components such as red blood cells are captured by the second blood cell separation member 4, while the liquid components without being captured are discharged from the second blood cell separation member 4.

Accordingly, the blood components such as red blood cells and white blood cells contained in the sample S are captured by the second blood cell separation member 4 as a filtering separating member and can thus not reach the judgment part 6 so that the judgment part 6 is not stained red by the color of red blood cells, and judgment is not made difficult.

In this embodiment, the first blood cell separation member 3 for separating blood cell components depending on their migration speed is disposed, and so liquid components initially infiltrate the second blood cell separation member 4 followed by infiltration of the blood cell components later than the liquid components. The second blood cell separation member 4 functions both in capturing the blood cells and in passing the liquid components so that the liquid components that have first infiltrated the second blood cell separation member 4 move smoothly in the second blood cell separation member 4 and are discharged as such. On the other hand, the blood cell components later than the liquid components infiltrate the second blood cell separation member 4, and after a majority of the liquid components are discharged, the blood cell components are captured by the second blood cell separation member 4. It follows that prior to passage of the liquid components, the blood cell components are captured by the second blood cell separation member 4, thereby passing the liquid components smoothly through the second blood cell separation member 4 while preventing the second blood cell separation member 4 from clogging with the blood cell components at an early stage.

The liquid components in the sample S that has been discharged from the second blood cell separation member 4 pass through the part of contact between the second blood cell separation member 4 and the chromatographic carrier 5 and moves to the chromatographic carrier 5.

As described above, the sample S can contain blood cell components of various sizes. Blood cell components (e.g., platelets) of relatively smaller size than the blood cell components such red blood cells and white blood cells contained in the sample S may not be captured in the second blood cell separation member 4, thus passing together with the liquid components through the member 4. However, even if platelets appear on the judgment part 6, judgment on the judgment part 6 is not made difficult by the color of platelets, and the influence of platelets on detection results is so low that even if platelets together with the liquid components have passed through the second blood cell separation 4, the working effect of this embodiment is not significantly influenced.

### (5) Judgment of examination results

The liquid components in the sample S that has transferred to the chromatographic carrier 5 move in the chromatographic carrier 5 by capillary phenomenon. The chromatographic carrier 5 carries, on the judgment part 6, a capturing substance capable of binding specifically to an intended analyte. Accordingly, when the liquid components have reached the judgment part 6, the capturing substance carried on the judgment part 6 is bound specifically to the analyte in the liquid components or to the complex of the analyte and the labeling substance, and then fixed on the judgment part 6. When the complex of the analyte and the labeling substance is fixed on the judgment part 6, the label of the labeling substance appears on the judgment part 6. For example, if the labeling substance is a substance labeled with colored particles, the color of the colored particles will appear on the judgment part 6, and it is thus confirmed that the analyte is present in the sample S. There can be both the case where after the analyte is bound to the labeling substance, the analyte is bound to the capturing substance and the case where after the analyte is bound to the capturing substance, the analyte is bound to the labeling substance.

The liquid components that have passed through the judgment part 6 are discharged from the chromatographic carrier 5 and absorbed into the absorbent member 7. The absorbent member 7 absorbs the liquid components thereby promoting the movement of the liquid components.

As described above, the test device 1 in accordance with this embodiment is constituted by including the label holding member 2 and the first blood cell separation member 3 in the order of passage of the sample S so that after addition to the sample addition part 10, the sample S while containing a sufficient amount of liquid components permeates into the label holding member 2, thereby sufficiently eluting the labeling substance held by the label holding member 2, and then move gradually to the first blood cell separation member 3.

Accordingly, the liquid components in the sample S discharged from the first blood cell separation member 3 contain the labeling substance at a uniform concentration with time without containing the labeling substance at an excessively high concentration. The sample S passing through the label holding member 2 may contain the labeling substance in the form of a mass, but such labeling substance will not appear on the chromatographic carrier 5, either by dispersion by physical resistance upon passing through the first blood cell separation member 3 and the second blood cell separation member 4 or by filtration with the second blood cell separation member 4.

Accordingly, the liquid components in the sample S moving in the chromatographic carrier 5 contains neither the labeling substance at an excessively high concentration nor the labeling substance in the form of a mass, and thus the liquid components in the sample S can smoothly move in the chromatographic carrier 5. It follows that according to the test device in this embodiment, the labeling substance is less likely to remain on the chromatographic carrier 5, and the background can thereby be reduced.

The "background" in this specification means the degree of nonspecific labeling on the chromatographic carrier excluding the judgment part, which is caused by the labeling substance remaining or retaining on the chromatographic carrier. For example, when the labeling substance is a colored substance, the background is the degree of coloration of the chromatographic carrier excluding the judgment part. It follows that when the labeling substance is a colored substance, high background means that the degree of coloration of the chromatographic carrier excluding the judgment part is high, and low background means that the degree of coloration of the chromatographic carrier excluding the judgment part is low.

According to the test device 1 in this embodiment, the concentration of the labeling substance contained in the sample S can be made uniform, and thus the development speed of the sample S can be increased to reduce the examination time.

In this embodiment, the label holding member 2 is constituted so as to have the sample addition part 10. By this structure, the amount of the sample S to be contacted per unit time with the label holding member 2 can be further increased, and the concentration of the labeling substance in the liquid components developed by the chromatographic carrier 5 can be made more uniform before and after elapsed time. The labeling substance-containing liquid components can thereby move more smoothly in the chromatographic carrier to further reduce the background.

Since the label holding member 2 is disposed in the position where the sample S is contacted first with the label holding member 2, the test device 1 in this embodiment can prolong the reaction time, that is, the time from a point when the sample S is contacted with the labeling substance till a point when the sample reaches the judgment part 6. The analyte in the liquid components contained in the sample S can thereby be sufficiently labeled to improve sensitivity.

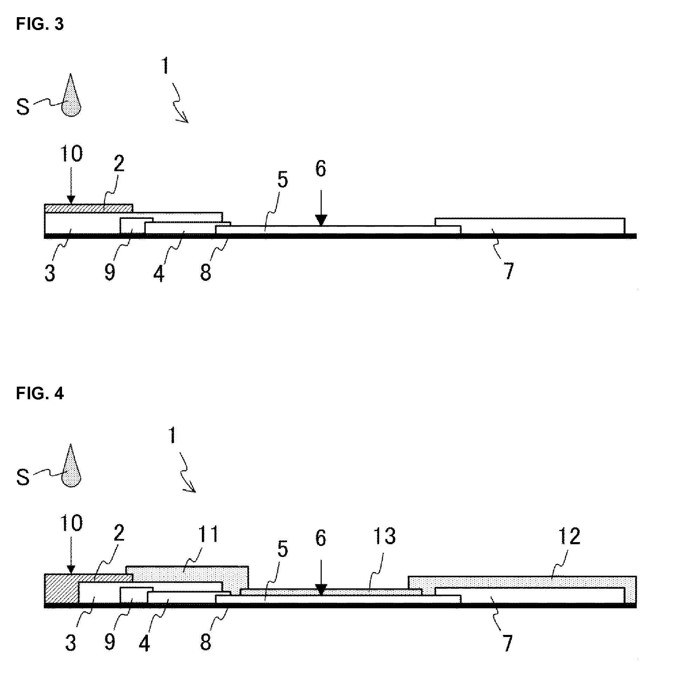

In the embodiment illustrated above, the test device is constituted such that it is provided with the protective sheets 11 to 13 for preventing the evaporation of liquid components in the sample S, but the protective sheets 11 to 13 may be omitted as shown in FIG. 3.

In the embodiment illustrated above, the test device is constituted such that the label holding member 2 and the first blood cell separation member 3 overlap vertically with each other in the vertical direction and are contacted with each other via the contact face 3a only in the vertical direction, but the test device of the present invention is not limited to such constitution. As shown in FIG. 4, the test device may be constituted such that the label holding member 2 and the first blood cell separation member 3 are contacted with each other both in the direction of sample development and in the vertical direction.

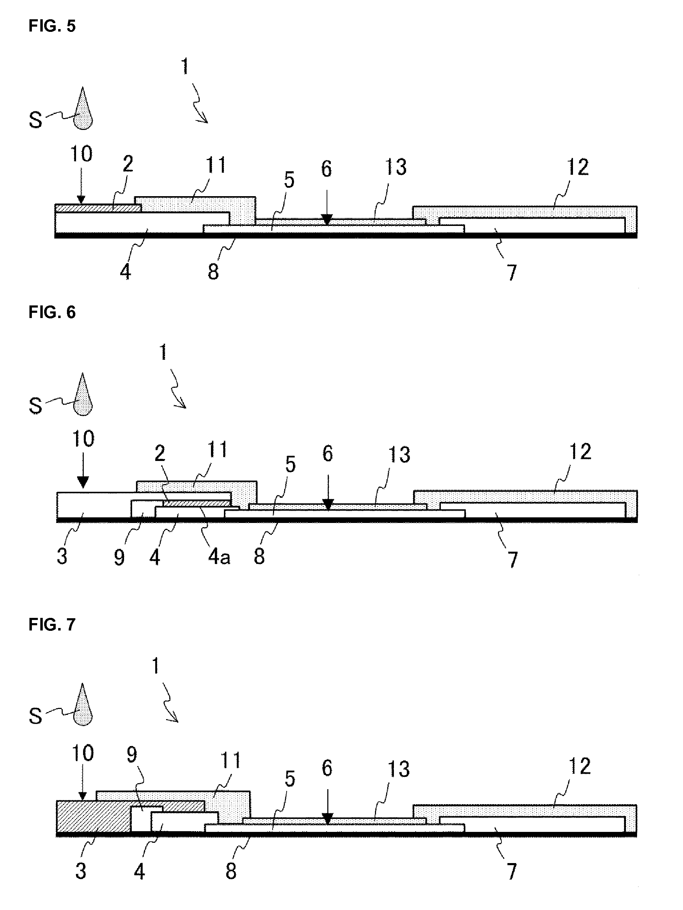

In the embodiment illustrated above, the test device is constituted such that the first blood cell separation member 3 and the second blood cell separation member 4 are used as the blood cell separation member for separating blood cell components in a sample, but the test device of the present invention is not limited to such constitution and may be constituted such that only one of the members is used. As shown in FIG. 5 for example, the test device of the present invention may be a device provided with only the second blood cell separation member 4 as the blood cell separation member. Even with this constitution given, the test device can prevent red blood cells from moving the chromatographic carrier 5, and so confirmation of judgment results in the judgment part 6 is not made difficult.

In the embodiment illustrated above, the test device is constituted such that it is provided with the label holding member 2, the first blood cell separation member 3 and the second blood cell separation member 4 in the order of sample passage, but the test device 1 is not limited to such constitution. For example, as shown in FIG. 6, the test device may be constituted such that the label holding member 2 is arranged between the first blood cell separation member 3 and the second blood cell separation member 4. For example as shown in FIG. 7, the first blood cell separation member 3 for holding the labeling substance may be used in place of the label holding member 2.

As described above, the first blood cell separation member 3 consists of a porous member having fine pores equal to, or slightly larger than, blood cells in size. Therefore, the predetermined blood components including red blood cells are prevented from moving by the first blood cell separation member 3, while the liquid components can move smoothly in the first blood cell separation member 3. That is, even given the constitution illustrated in FIG. 6 or 7, it is possible to contact, with the labeling substance, liquid components in such a sufficient amount that the concentration of the labeling substance in the liquid components discharged into the chromatographic carrier 5 can be made uniform with time, thereby sufficiently reducing the background.

If the test device is constituted so as to allow a sample to pass through the blood cell separation part and the label holding member in this order such as in the conventional chromatographic test device, then the added sample will be transferred little by little from the blood cell separation part to the label holding member by capillary phenomenon, to elute the labeling substance, and will be simultaneously developed on the test device. Consequently, the concentration of the labeling substance in the sample discharged into the chromatographic carrier is excessively high upon initial discharge of the sample into the chromatographic carrier and is then gradually lowered as the sample is discharged into the chromatographic carrier, thus becoming uneven with time. In addition, the sample that has passed the label holding member is discharged as it is into the chromatographic carrier so that the labeling substance dissolved in the sample is discharged, without being sufficiently dispersed, into the chromatographic carrier. Accordingly, the sample to be discharged initially into the chromatographic carrier contains a high concentration of the labeling substance in a state not sufficiently dispersed, and thus the sample cannot move smoothly on the chromatographic carrier so that the labeling substance sometimes remains or retains on the chromatographic carrier to cause an increase in background.

In the chromatographic test device shown for example in FIG. 1, on the other hand, the sample added will, prior to contacting with the blood cell separation member, contact with the label holding member so that the amount of the sample contacted per unit time with the labeling holding member can be increased. Accordingly, the concentration of the labeling substance in the sample discharged into the chromatographic carrier can be made uniform with time, and the concentration of the labeling substance in the sample is not made excessively high. Further, the labeling substance is eluted into the sample and then passes through the blood cell separation member, and thus the labeling substance is sufficiently dispersed in the sample by physical resistance exerted on it upon passing through the blood cell separation member. Accordingly, the sample can move smoothly in the chromatographic carrier, thus reducing the amount of the labeling substance remaining or retaining on the chromatographic carrier, to reduce the background.

### Examples

### 1. Preparation of a test device in Example 1

As shown in FIG. 1, the test device in Example 1 was prepared. Specifications for members are as shown in Table 1. Overlapping among the members is as shown in FIG. 1. As shown in FIG. 1, the test device in Example 1 is constituted by including a label holding member 2, a first blood cell separation member 3, a second blood cell separation member 4, a chromatographic carrier 5 and an absorbent member 7 in the order of passage of a sample. The label holding member 2 has a sample addition part 10 to which sample S is added.

**Table 1**

| Constitution of the test device in Example 1 | | | |
|---|---|---|---|
| Name | Size | Type | Manufacturer |
| Label holding member 2 | 10 mm × 5 mm | 8975 | PALL |
| First blood cell separation member 3 | 20 mm × 5 mm | MF1 | Whatman |
| Second blood cell separation member 4 | 10 mm × 5 mm | BTS SP300 | PALL |
| Sealing member 9 | 10 mm × 5 mm | 7759 | Adhesives |
| Chromatographic carrier 5 | 30 mm × 5 mm | SHF 13504 | Millipore Corporation |
| Substrate 8 | 100 mm | 9020 | Adhesives |
| Protective sheet 11 | 20 mm × 5 mm | 7759 | Adhesives |
| Protective sheet 12 | 40 mm × 5 mm | 7759 | Adhesives |
| Protective sheet 13 | 40 mm × 5 mm | 7759 | Adhesives |
| Absorbent member 7 | 35 mm × 5 mm | CF5 | Whatman |

### 2. Preparation of a test device in Comparative Example 1

As shown in FIG. 2, the test device in Comparative Example 1 was prepared. The test device in Comparative Example 1, unlike the test device in Example 1, is constituted by including a first blood cell separation member 3, a second blood cell separation member 4 and a label holding member 2 in the order of passage of a sample. In conformity to the change in the position of the label holding member 2, a sample addition part 10 to which a sample is added in the test device in Comparative Example 1 is disposed in the first blood cell separation member 3. Except for these features, the test device in Comparative Example 1 has the same constitution as in the test device in Example 1.

### 3. Test for confirmation of effect

Then, a test for demonstrating the effect of the present invention was carried out by the following method.

### 3-1. Confirmation of effect on reduction of background

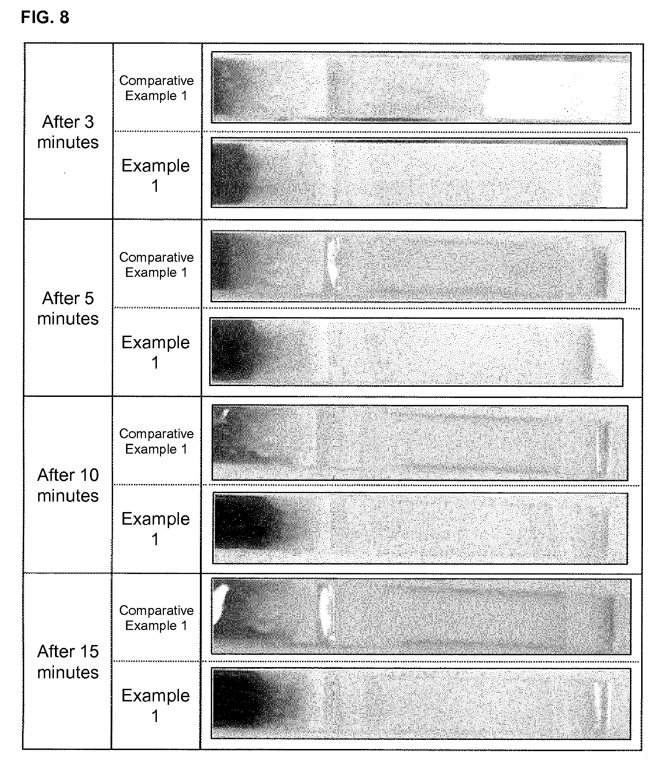

The test device in Example 1 and the test device in Comparative Example 1 were used, and 100 µL of whole blood was added to each of their sample addition parts and then developed on each of the test devices 1, and a background appearing on the chromatographic carrier 5 was observed. The result is shown in FIG. 8. FIG. 8 is a photograph showing the development state of the sample with time in each of the test devices in Example 1 and Comparative Example 1.

As can also be evident from FIG. 8, the labeling substance remains less on the chromatographic carrier 5 in the test device in Example 1 than in the test device in Comparative Example 1, at all time points, that is, 3 minutes, 5 minutes, 10 minutes and 15 minutes after addition of the sample. Particularly 5 minutes or more after addition of the sample, the remaining labeling substance on the chromatographic carrier 5 is at such a level as not to be confirmed with the naked eye, and the background is reduced to an infinitely low level in the test device in Example 1.

In the test device in Comparative Example 1, on the other hand, the labeling substance remains on the chromatographic carrier 5 at any time points, that is, 3 minutes, 5 minutes, 10 minutes and 15 minutes after addition of the sample, and the background is higher than that in Example 1.

From this result, it was demonstrated that when whole blood was examined with the test device in Example 1, the labeling substance remaining on the chromatographic carrier was decreased, and the background was reduced.

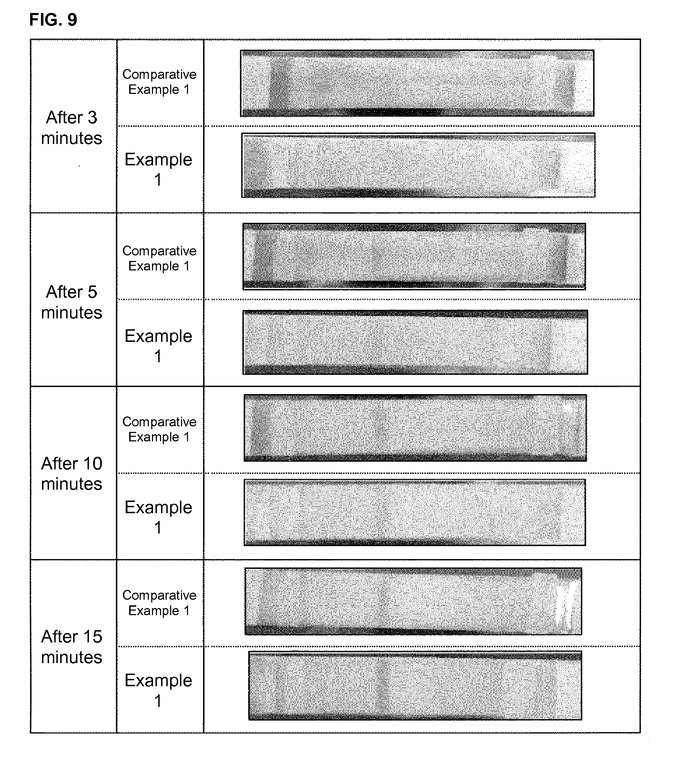

In the same manner as in the above experiment, the test device in Example 1 and the test device in Comparative Example 1 were used, and 100 µL of serum containing an HBs antigen was added to each of their sample addition parts and then developed on each of the test devices 1, and a background appearing on the chromatographic carrier 5 was observed. A substance being capable of binding specifically to the HBs antigen and being labeled with a labeling substance was contained in the label holding member 2 in each of the devices in Example 1 and Comparative Example 1, and a substance capable of binding specifically to the HBs antigen was carried on each judgment part 6. The result is shown in FIG. 9. FIG. 9 is a photograph showing the development state of the sample with time in each of the test devices in Example 1 and Comparative Example 1.

As is also evident from FIG. 9, it can be seen that the labeling substance remains less on the chromatographic carrier 5 in the test device in Example 1 than that in the test device in Comparative Example 1, at any time points, that is, 3 minutes, 5 minutes and 10 minutes after addition of the sample, and that the background is reduced in the test device in Example 1.

In the test device in Comparative Example 1, on the other hand, the labeling substance remains in a large amount on the chromatographic carrier 5 at any time points, that is, 3 minutes, 5 minutes and 10 minutes after addition of the sample, and the background is higher than that in Example 1. At the time point 15 minutes after addition, there is no significant difference in background between Comparative Example 1 and Example 1, but as can be seen from FIG. 9, the judgment part 6 in Example 1 is darker and more vivid than the judgment part 6 in Comparative Example 1.

From this result, it was demonstrated that when serum was examined with the test device in Example 1, the labeling substance remaining on the chromatographic carrier was decreased, and the background was reduced.

From the foregoing, the test device in Example 1 can reduce the background irrespective of the type of sample, that is, regardless of whether the sample is whole blood or serum. Accordingly, it was demonstrated that more accurate examination with low possibility of erroneous judgment became possible by using the chromatographic test device in Example 1.

### 3-2. Confirmation of effect on development speed

Then, the following experiment was carried out to confirm the development speed when the test device in this example was used.

The test device in Example 1 and the test device in Comparative Example 1 were used, and 100 µL of whole sample was added to their sample addition parts and developed on each of the test devices 1, and the time required for the sample after addition to appear on the chromatographic carrier 5, and the time required for the sample after addition to reach the absorbent member 7, were measured. This experiment was conducted for 3 times each using whole blood collected from a different living body. The results are shown in Tables 2-1 and 2-2.

**Table 2-1**

| Time (sec) elapsed until appearance on the chromatographic carrier | | |
|---|---|---|
| | Test Device in Example 1 | Test Device in Comparative Example 1 |
| Sample 1 | 19 | 132 |
| Sample 2 | 24 | 108 |
| Sample 3 | 18 | 138 |

**Table 2-2**

| Time (sec) elapsed until arrival at the absorbent member | | |
|---|---|---|
| | Test Device in Example 1 | Test Device in Comparative Example 1 |
| Sample 1 | 189 | 391 |
| Sample 2 | 178 | 269 |
| Sample 3 | 141 | 292 |

From Tables 2-1 and 2-2, it can be seen that when the test device in Example 1 was used, both the time required for the added sample to appear on the chromatographic carrier 5 and the time required for the added sample to reach the absorbent member 7 were shorter than with the test device in Comparative Example 1.

In the same manner as described above, the test device in Example 1 and the test device in Comparative Example 1 were used, and 100 µL of serum was added to each of their sample addition parts and then developed on each of the test devices 1, and the time required for the sample after addition to appear on the chromatographic carrier 5, and the time required for the sample after addition to reach the absorbent member 7, were measured. This experiment was conducted for 3 times each using serum collected from a different living body. The results are shown in Tables 3-1 and 3-2.

**Table 3-1**

| Time (sec) elapsed until appearance on the chromatographic carrier | | |
|---|---|---|
| | Test Device in Example 1 | Test Device in Comparative Example 1 |
| Sample 4 | 17 | 10 |
| Sample 5 | 11 | 17 |
| Sample 6 | 13 | 14 |

**Table 3-2**

| Time (sec) elapsed until arrival at the absorbent member | | |
|---|---|---|
| | Test Device in Example 1 | Test Device in Comparative Example 1 |
| Analyte 4 | 88 | 109 |
| Analyte 5 | 119 | 158 |
| Analyte 6 | 139 | 152 |

From Tables 3-1 and 3-2, it can be seen that when the test device in Example 1 was used, both the time required for the added sample to appear on the chromatographic carrier 5 and the time required for the added sample to reach the absorbent member 7 were shorter than with the test device in Comparative Example 1.

From the foregoing, the test device in Example 1 can increase the development speed of the sample irrespective of sample type, that is, regardless of whether the sample is whole blood or serum. Accordingly, it was demonstrated that more accurate examination became possible by using the chromatographic test device in Example 1.

## Claims

1. A chromatographic test device comprising, in the order of passage of a sample containing blood cell components and liquid components:
a label holding member provided with a labeling substance capable of binding specifically to an analyte contained in the liquid components,
a blood cell separation member for separating predetermined blood cell components in the sample from the liquid components, and
a chromatographic carrier carrying a capturing substance capable of binding specifically to the analyte.

2. The chromatographic test device according to claim 1, wherein the label holding member is provided with a sample addition part to which a sample is added.

3. The chromatographic test device according to claim 1 or 2, wherein the label holding member and the blood cell separation member each consist of a porous member, and the pore size of the label holding member is larger than the pore size of the blood cell separation member.

4. The chromatographic test device according to any one of claims 1 to 3, wherein the label holding member and the blood cell separation member are disposed so as to overlap with each other in the vertical direction in a horizontally disposed state of the test device.

5. The chromatographic test device according to any one of claims 1 to 4, wherein the blood cell separation member comprises, in the order of sample passage:
a first blood cell separation member that separates predetermined blood cell components from liquid components by moving the liquid components faster than the predetermined blood cell components, and
a second blood cell separation member that separates predetermined blood components from liquid components by capturing the predetermined blood components and passing the liquid components.

6. The chromatographic test device according to any one of claims 1 to 5, which further comprises an absorbent member for absorbing the liquid components in the sample that has passed through the chromatographic carrier.

7. The chromatographic test device according to any one of claims 1 to 6, which further comprises a protective member for preventing evaporation of the liquid components in the sample.

8. A chromatographic test device comprising, in the order of passage of a sample containing blood cell components and liquid components:
a first blood cell separation member for separating predetermined blood cell components in the sample from the liquid components,
a label holding member provided with a labeling substance capable of binding specifically to an analyte contained in the liquid components,
a second blood cell separation member for further separating, from the liquid components, the blood cell components separated by the first blood cell separation member, and
a chromatographic carrier carrying a capturing substance capable of binding specifically to the analyte.

9. The chromatographic test device according to claim 8, wherein the first blood cell separation member is a member that separates predetermined blood cell components from liquid components by moving the liquid components faster than the predetermined blood cell components.

10. The chromatographic test device according to claim 8 or 9, wherein the first blood cell separation member consists of a porous member.

11. A chromatographic test device comprising, in the order of passage of a sample containing blood cell components and liquid components:
a first blood cell separation member for separating predetermined blood cell components from the liquid components, the first blood cell separation member being provided with a labeling substance capable of binding specifically to an analyte contained in the liquid components,
a second blood cell separation member for further separating, from the liquid components, the blood cell components separated by the first blood cell separation member, and
a chromatographic carrier carrying a capturing substance capable of binding specifically to the analyte.

12. The chromatographic test device according to claim 11, wherein the first blood cell separation member is provided with a sample addition part to which a sample is added.

13. The chromatographic test device according to any one of claims 8 to 10, wherein the first blood cell separation member and the second blood cell separation member each consist of a porous member, and the pore size of the label holding member is larger than the pore size of the first blood cell separation member.
